(19) Europäisches Patentamt
European Patent Office
Office européen des brevets

(11) **EP 2 111 544 B1**

(12) **FASCICULE DE BREVET EUROPEEN**

(45) Date de publication et mention de la délivrance du brevet:
**12.12.2018 Bulletin 2018/50**

(21) Numéro de dépôt: **08762049.8**

(22) Date de dépôt: **08.02.2008**

(51) Int Cl.:
*G01N 25/08* (2006.01)    *G01N 25/10* (2006.01)
*G01N 25/14* (2006.01)    *G01N 33/28* (2006.01)

(86) Numéro de dépôt international:
**PCT/FR2008/050192**

(87) Numéro de publication internationale:
**WO 2008/104685 (04.09.2008 Gazette 2008/36)**

(54) **PROCEDE DE DISTILLATION AUTOMATIQUE D'ECHANTILLONS LIQUIDES SOUS PRESSION ATMOSPHERIQUE DANS UN APPAREIL DE DISTILLATION NORMALISE**

VERFAHREN ZUR AUTOMATISCHEN DESTILLATION FLÜSSIGER PROBEN UNTER ATMOSPHÄRENDRUCK IN EINER STANDARDISIERTEN DESTILLATIONSVORRICHTUNG

METHOD FOR AUTOMATICALLY DISTILLING LIQUID SPECIMENS AT ATMOSPHERIC PRESSURE IN A STANDARDIZED DISTILLATION APPARATUS

(84) Etats contractants désignés:
**AT BE BG CH CY CZ DE DK EE ES FI FR GB GR HR HU IE IS IT LI LT LU LV MC MT NL NO PL PT RO SE SI SK TR**

(30) Priorité: **13.02.2007 FR 0753218**

(43) Date de publication de la demande:
**28.10.2009 Bulletin 2009/44**

(73) Titulaire: **Instrumentation Scientifique de Laboratoire ISL**
**14790 Verson (FR)**

(72) Inventeurs:
• **URVANTSAU, Viachaslau**
**F-14120 Mondeville (FR)**
• **CLERIS, Hervé**
**F-14220 Curcy sur Orne (FR)**

(74) Mandataire: **Cabinet HERRBURGER**
**115, Boulevard Haussmann**
**75008 Paris (FR)**

(56) Documents cités:
**FR-A1- 2 410 818**    **FR-A1- 2 815 413**
**GB-A- 2 122 386**    **US-A- 3 364 731**
**US-A- 4 250 739**

• **SPIEKSMA W: "Prediction of ASTM Method D86 Distillation of Gasolines and Naphtas according to the Fugacity-Filmmodel from Gas Chromatographic Detailed Hydrocarbon Analysis" JOURNAL OF CHROMATOGRAPHIC SCIENCE, PRESTON PUBLICATIONS, NILES, IL, US, vol. 36, no. 9, 1 septembre 1998 (1998-09-01), pages 467-475, XP002100180 ISSN: 0021-9665**

**Description**

La présente invention a pour objet un procédé de distillation automatique d'échantillons liquides, en particulier d'échantillons de produits pétroliers sous pression atmosphérique dans un appareil de distillation normalisé.

Il est connu que les caractéristiques de distillation des produits pétroliers sont représentatives des performances de ces produits ainsi que des risques qu'ils peuvent faire encourir à leurs utilisateurs.

La détermination de ces caractéristiques présente notamment une grande importance dans le cas de carburants destinés à l'industrie automobile ou à l'aviation où les problèmes liés à la sécurité sont primordiaux.

Ces caractéristiques sont en particulier des tables ou des courbes représentant le pourcentage d'un échantillon évaporé selon la température pendant une distillation ou encore le volume du résidu et les pertes.

Les spécialistes peuvent déduire de ces caractéristiques quel sera le comportement d'un produit pétrolier donné dans une situation donnée et donc déterminer si ce produit peut ou non être utilisé en toute sécurité, ce de manière à permettre d'obtenir des performances recherchées.

Dans ce contexte, les spécialistes ont édicté différentes normes qui définissent très précisément les conditions dans lesquelles doivent être obtenues de telles caractéristiques de distillation.

Par suite, pour donner des résultats exploitables, les distillations doivent être mises en oeuvre en respectant scrupuleusement ces normes.

Il existe actuellement sur le marché différents appareils de distillation automatique permettant d'effectuer la mesure des paramètres de distillation d'un échantillon inconnu en respectant ces normes.

Ces appareils de distillation normalisés renferment en règle générale :

- un élément chauffant,
- un ballon de distillation dont le col peut être fermé par un bouchon étanche muni d'un thermomètre permettant de mesurer la température des vapeurs évaporées et peut être connecté à un condenseur,
- un cylindre collecteur permettant de recueillir le condensat et équipé d'organes de mesure de la quantité de condensat ainsi recueillie en fonction du temps, et
- des moyens de commande et de régulation permettant de commander et de faire varier dans le temps une grandeur de fonctionnement de l'élément chauffant, notamment la température ou la puissance électrique de cet élément de manière à obtenir des paramètres de distillation, en particulier des vitesses de distillation et/ou des temps pour des volumes particuliers conformément à une norme prédéfinie.

Les normes imposent en particulier, pour un groupe déterminé de produits, des paramètres tels que le temps écoulé entre le début du chauffage de l'échantillon et l'obtention du point d'ébullition initial IBP (Initial Boiling Point) c'est-à-dire l'instant pour lequel la première goutte de condensat est observée dans le cylindre collecteur, ou encore le temps écoulé entre l'IBP et l'obtention du point de distillation à 5 %, c'est-à-dire du point pour lequel 5 % du volume initial de l'échantillon ont été recueillis dans le cylindre collecteur.

Ces normes imposent également la vitesse de distillation entre le point de distillation à 5 % et le point pour lequel il ne reste plus que 5 ml d'échantillon dans le ballon de distillation (c'est-à-dire le volume d'échantillon évaporé ou condensé par unité de temps au cours de la distillation) ou encore le temps écoulé entre le point pour lequel il ne reste plus que 5 ml d'échantillon dans le ballon de distillation et le point d'ébullition final FBP (Final Boiling Point) c'est-à-dire la fin de la distillation.

Les appareils de distillation automatique fonctionnant conformément à ces normes actuellement proposés sur le marché mettent en oeuvre un procédé selon lequel :

- on introduit une quantité prédéfinie d'un échantillon à analyser dans le ballon de distillation,
- on positionne ce ballon de distillation sur l'élément chauffant, on le ferme et on le connecte au condenseur,
- on classe l'échantillon à analyser dans un groupe défini par la norme choisie, et
- on lance la distillation de l'échantillon à analyser en mesurant constamment la quantité de condensat recueillie dans le cylindre collecteur, la température des vapeurs évaporées, ainsi que la grandeur de fonctionnement de l'élément chauffant, notamment la température ou la puissance électrique de cet élément, et
- on transmet les valeurs ainsi mesurées aux moyens de commande et de régulation qui commandent en retour la grandeur de fonctionnement de l'élément chauffant de manière à obtenir directement et automatiquement des paramètres de distillation, en particulier des vitesses de distillation et/ou des temps pour des volumes particuliers conformément à la norme choisie.

De tels appareils de distillation présentent un certain nombre d'inconvénients, notamment liés à l'inertie entre le point de mesure de la température des vapeurs évaporées et la récupération dans le cylindre collecteur des vapeurs condensées dont on a mesuré la température.

**EP 2 111 544 B1**

Le principal inconvénient des appareils de distillation classiques est toutefois lié à la nécessité d'effectuer différentes mesures empiriques et par tâtonnement avant la distillation proprement dite, c'est-à-dire la détermination des caractéristiques de distillation d'un échantillon inconnu.

De telles mesures préliminaires sont en effet particulièrement longues et présentent en outre l'inconvénient d'exiger un volume important d'échantillon qui n'est pas toujours disponible ; de plus ces mesures sont largement tributaires de l'habileté de l'opérateur.

Il est en effet nécessaire de sélectionner préalablement un groupe dans lequel peut être classé l'échantillon inconnu parmi les groupes définis par la norme choisie, ce groupe imposant une température de début de distillation, et de prévoir quel sera le point IBP ainsi que le point pour lequel il ne restera plus que 5 % de l'échantillon à analyser dans le ballon de distillation.

Les points ainsi prévus doivent en effet être introduits dans l'appareil avant la distillation pour permettre aux moyens de commande et de régulation de déterminer différents paliers de chauffage de l'échantillon, variables au cours du temps et de commander la grandeur de fonctionnement de l'élément chauffant en conséquence, de façon à pouvoir obtenir les conditions de distillation dans les limites imposées par la norme choisie.

Or, ces prévisions s'avèrent souvent inexactes, ce qui oblige l'opérateur à effectuer plusieurs essais avant de pouvoir obtenir les conditions de distillation dans les limites imposées par la norme choisie ce qui entraîne une perte de temps pouvant être importante, et surtout une perte d'échantillon qui peut dans certains cas n'être disponible qu'en quantité limitée.

Le document US 3 364 731 décrit un procédé de distillation automatique des produits pétroliers selon le norme ASTM D 86.

La présente invention a pour objet de proposer un procédé de distillation automatique d'échantillons liquides, en particulier d'échantillons de produits pétroliers sous pression atmosphérique dans un appareil de distillation normalisé permettant de s'affranchir de ces différentes contraintes et d'effectuer directement et sans essais préalables une distillation en respectant une norme, ce, en n'introduisant préalablement dans l'appareil qu'un seul paramètre, à savoir le groupe dans lequel se situe l'échantillon à analyser, parmi la série de groupes définis par la norme choisie.

Selon l'invention, ce procédé est caractérisé en ce que :

- on introduit une quantité prédéfinie d'un échantillon à analyser dans le ballon de distillation d'un appareil de distillation normalisé,
- on positionne ce ballon de distillation sur l'élément chauffant, on le ferme et on le branche sur le condenseur,
- on classe l'échantillon à analyser dans l'un des groupes définis par la norme choisie, et
- on lance la distillation de l'échantillon à analyser en mesurant constamment la quantité de condensat recueillie dans le cylindre collecteur, la température des vapeurs évaporées, la température de l'échantillon liquide présent dans le ballon de distillation, ainsi que la grandeur de fonctionnement de l'élément chauffant, notamment la température ou la puissance électrique de cet élément, et
- on transmet les valeurs ainsi mesurées aux moyens de commande et de régulation qui commandent en retour la grandeur de fonctionnement de l'élément chauffant de manière à obtenir directement et automatiquement des caractéristiques et/ou des courbes de distillation conformément à la norme choisie.

Selon l'invention, les caractéristiques de distillation de l'échantillon à analyser sont le point d'ébullition initial (IBP) et/ou le point de distillation à 5 % et/ou le point pour lequel il ne reste que 5 % de l'échantillon à analyser dans le ballon de distillation.

Ce procédé se distingue ainsi du procédé classiquement mis en oeuvre lors de l'utilisation des appareils de distillation normalisés classiques par le fait que l'on mesure constamment la température de l'échantillon liquide présent dans le ballon de distillation.

Cette mesure de la température de l'échantillon liquide est en règle générale une mesure indirecte ; en effet les normes actuellement en vigueur interdisent toute mesure directe de cette température.

La mise en oeuvre du procédé conforme à l'invention permet à l'appareil de distillation normalisé de déterminer automatiquement :

- le point IBP, le point de distillation à 5 % et le point pour lequel il ne reste que 5 % de l'échantillon à analyser dans le ballon de distillation, ce dans les conditions imposées par la norme choisie,
- la grandeur de fonctionnement de l'élément chauffant avant le point IBP et entre le point IBP et le point de distillation à 5 % pour obtenir ces points dans les conditions imposées par la norme choisie,
- la grandeur de fonctionnement de l'élément chauffant en cours de distillation entre le point de distillation à 5 % et le point pour lequel il ne reste que 5 % de l'échantillon à analyser dans le ballon de distillation en corrigeant automatiquement cette grandeur pour obtenir une vitesse de distillation correcte tel qu'imposée par la norme choisie, et

3

- la grandeur de fonctionnement de l'élément chauffant entre le point pour lequel il ne reste que 5 % de l'échantillon à analyser dans le bal-lon de distillation et le point d'ébullition final FBP de manière à obtenir ce point dans les conditions imposées par la norme choisie.

Le procédé conforme à l'invention permet en outre de prévoir le résidu dans le ballon de distillation et les pertes, à partir des caractéristiques de distillation de l'échantillon.

Selon une caractéristique préférentielle de l'invention, on mesure indirectement la température de l'échantillon liquide présent dans le ballon de distillation au moyen d'un capteur infrarouge.

L'utilisation d'un tel capteur s'est en effet avérée particulièrement avantageuse.

Selon l'invention, on a ainsi comparé les temps de réponse d'un thermocouple plongeant directement dans l'échantillon liquide présent dans le ballon de distillation et d'un capteur infrarouge permettant une mesure indirecte de la température de cet échantillon.

On a ainsi pu vérifier que la mise en oeuvre d'un capteur infrarouge permet d'obtenir une indication suffisamment fiable de la température de l'échantillon liquide présent dans le ballon de distillation.

Toutefois pour pouvoir utiliser valablement un tel capteur infrarouge de façon à obtenir une évaluation correcte de la température de l'échantillon liquide présent dans le ballon de distillation, il est nécessaire de bloquer le rayonnement infrarouge émis par l'élément chauffant.

Selon une autre caractéristique de l'invention, on interpose à cet effet entre l'élément chauffant et le ballon de distillation une plaque isolante perforée imperméable au rayonnement infrarouge.

La présence d'une telle plaque est en fait indispensable.

Conformément à l'invention, celle-ci peut avantageusement être réalisée en une céramique à base de silicate de calcium synthétique comprimé telle qu'à titre d'exemple le produit commercialisé par la Société ELIT sous la dénomination DURATEC 1000.

Compte tenu de ce qui précède, le procédé conforme à l'invention a pour avantage essentiel de permettre de s'affranchir de la prévision préalable du point IBP d'un échantillon inconnu grâce à des mesures effectuées sur l'échantillon pendant la période précédant ce point qui était auparavant « occultée ».

Ces mesures permettent en effet d'obtenir directement le point IBP après un temps de chauffage correspondant à celui imposé par la norme choisie suite à l'introduction préalable dans l'appareil d'une seule information, à savoir le groupe auquel appartient l'échantillon à analyser selon cette norme.

Selon une caractéristique non limitative de l'invention, la norme choisie est la norme ASTM D 86.

Dans l'exemple particulier de cette norme, on a effectué des tests préliminaires selon lesquels on a analysé soixante quatre produits connus couvrant les quatre groupes.

Ces produits ont été sélectionnés de façon à obtenir les plages de température et les pentes de distillation les plus larges possibles pour tous les points caractéristiques de la distillation.

On a ainsi dressé le tableau 1 qui indique la température minimum et la température maximum du produit liquide au point IBP pour les produits appartenant à chacun des quatre groupes ainsi que les temps minimum et maximum permettant d'obtenir ce point conformément à la norme.

Tableau 1

| Groupe | Minimum | | Maximum | |
|---|---|---|---|---|
| | Température du produit liquide au point IBP | Temps entre le début du chauffage et le point IBP | Température du produit liquide au point IBP | Temps entre le début du chauffage et le point IBP |
| 1 | 36 | 300 | 95 | 600 |
| 2 | 76 | 300 | 211 | 600 |
| 3 | 36 | 300 | 95 | 600 |
| 4 | 182 | 300 | 327 | 900 |

Ce tableau prouve à titre d'exemple que pour les produits du groupe 1 à faible température d'ébullition, la température du liquide présent dans le ballon de distillation au point IBP est de l'ordre de 36°C et est obtenue environ 300 secondes c'est-à-dire 5 minutes après le début du chauffage alors que pour les produits de ce même groupe à température d'ébullition élevée, la température du liquide présent dans le ballon de distillation au point IBP est de l'ordre de 95°C et est obtenue environ 600 secondes c'est-à-dire 10 minutes après le début du chauffage.

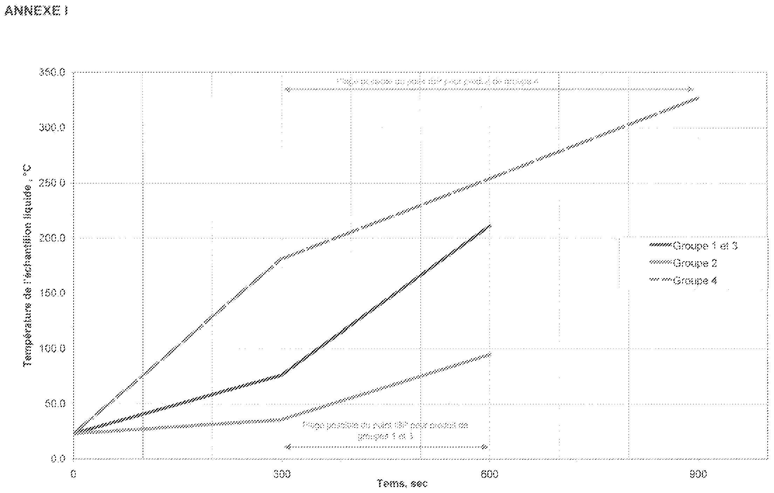

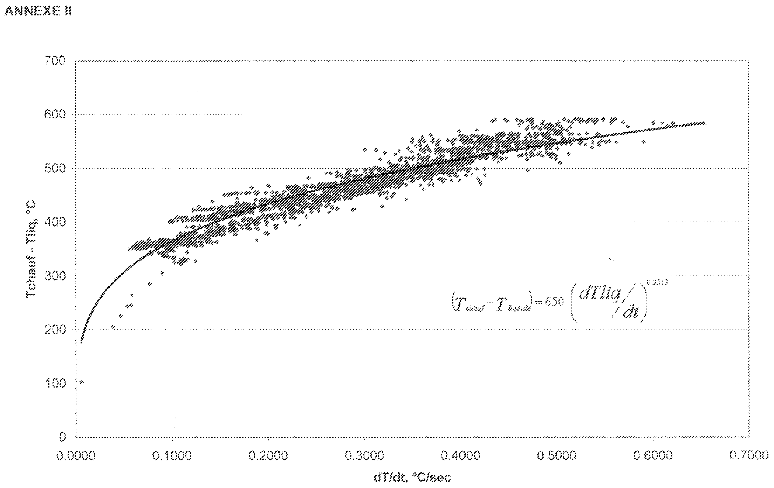

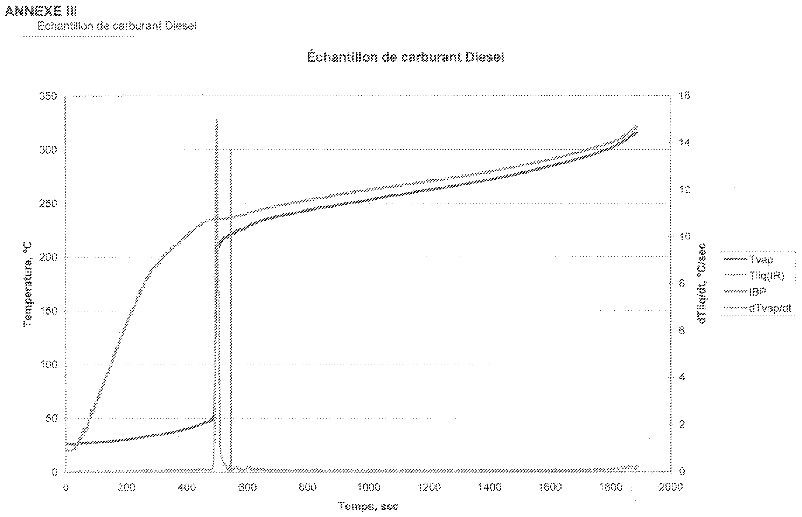

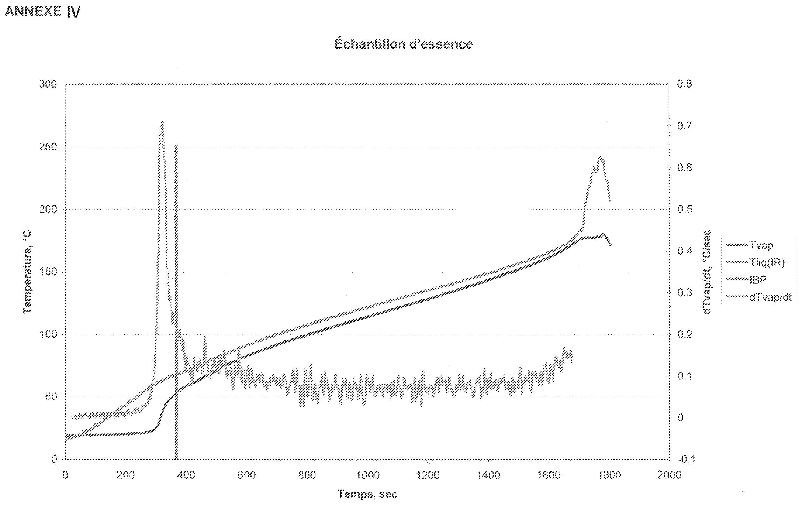

Il est par suite nécessaire de réguler les variations de la température de l'échantillon liquide présent dans le ballon de distillation en fonction du temps de la manière représentée sur la figure jointe en annexe 1.

Selon l'invention, cette régulation de la température de l'échantillon liquide présent dans le ballon de distillation a pu être obtenue en équipant un appareil de distillation normalisé d'un capteur infrarouge.

Pour obtenir des courbes représentant les variations de la température de l'échantillon liquide présent dans le ballon de distillation en fonction du temps correspondant à celle figurant en annexe 1, il est nécessaire de faire varier une grandeur de fonctionnement de l'élément chauffant en tenant compte des propriétés dynamiques et statiques de cet élément, c'est-à-dire des variations de température et des déperditions d'énergie calorifique de celui-ci, ce de façon à maîtriser l'énergie calorifique effectivement transmise à l'échantillon liquide présent dans le ballon de distillation.

On a considéré qu'une bonne approximation de cette énergie est donnée par la différence existant entre la température $T_{chauf}$ de l'élément chauffant mesurée en continu par un thermocouple et la température $T_{liq}$ de l'échantillon liquide présent dans le ballon de distillation mesurée en continu au moyen du capteur infrarouge.

Il a ainsi été proposé conformément à l'invention de réguler le chauffage de l'élément chauffant à partir de la température de l'échantillon liquide présent dans le ballon de distillation.

Pour établir la relation existant entre ces différents paramètres de la régulation, on a chauffé des produits tests avec des puissances de chauffage différentes de façon à dresser la courbe représentée sur la figure jointe en annexe 2 représentant les variations de la différence entre la température $T_{chauf}$ de l'élément chauffant et la température $T_{liq}$ de l'échantillon liquide en fonction des variations dTliq/dt de la température de cet échantillon liquide en fonction du temps.

On a ainsi pu établir que la différence entre la température $T_{chauf}$ de l'élément chauffant et la température $T_{liq}$ de l'échantillon liquide présent dans le ballon de distillation en fonction des variations dTliq/dt de la température de l'échantillon liquide en fonction du temps était représentée par l'équation 1 ci-dessous.

$$T_{chauf} - T_{liq} = A \cdot \left( \frac{T_{liq}}{dt} \right)^{B} \quad \text{(I)}$$

dans laquelle A et B sont des coefficients empiriques.

Dans le cas particulier de l'élément chauffant utilisé, ces coefficients avaient les valeurs suivantes : A = 650 et B = 0.2513.

Par suite, et selon une autre caractéristique de l'invention, conformément à la norme ASTM D 86, avant le point IBP, on régule la température $T_{chauf}$ de l'élément chauffant à partir de la température $T_{liq}$ de l'échantillon liquide présent dans le ballon de distillation conformément à la formule

$$T_{chauf} - T_{liq} = A \cdot \left( \frac{T_{liq}}{dt} \right)^{B}$$

dans laquelle dTliq/dt représentent les variations de la température de l'échantillon liquide en fonction du temps fourni par les courbes en annexe 1.

L'utilisation de cette formule permet en effet d'effectuer la régulation de la température de l'élément chauffant au début de la distillation, avant le point IBP, conformément à la norme ASTM D 86 et avec une erreur minimum.

On a toutefois pu établir, à partir de tests de distillation effectués sur des échantillons de produits commerciaux de type essence, diesel, kérosène que si la mise en oeuvre d'une régulation de l'élément chauffant en utilisant la formule I permet d'obtenir un point IBP correct conformément à la norme, elle ne permet pas d'obtenir un point de distillation à 5 % correct.

Il s'est en effet avéré que, dans ce cas, l'énergie calorifique transmise à l'échantillon liquide est notablement trop élevée, cette différence étant plus importante pour des produits purs ou presque purs.

En conséquence, il a été considéré que la dernière régulation conformément à la formule I devait de préférence être effectuée avant l'obtention du point IBP, c'est-à-dire plusieurs secondes avant l'obtention de la première goutte de condensat dans le cylindre collecteur, ce qui implique la détermination du début du processus d'ébullition de l'échantillon liquide présent dans le ballon de distillation.

Conformément à l'invention, il a été proposé à cet effet de prendre en compte la variation brusque de la température mesurée par le thermomètre vapeur équipant le ballon de distillation.

Pour vérifier l'opportunité d'une telle prise en compte, on a tracé d'une part dans le cas d'un échantillon de carburant diesel et d'autre part dans le cas d'une essence des courbes représentant les variations en fonction du temps :

- de la température des vapeurs évaporées mesurée par le thermomètre vapeur,
- de la température de l'échantillon liquide présent dans le ballon de distillation mesurée par le capteur infrarouge,
- des variations en fonction du temps de la température des vapeurs mesurée par le thermomètre vapeur dTvap/dt.

et on a représenté le point IBP sur ces courbes.

Ces courbes sont respectivement rassemblées en annexes 3 et 4.

Ces courbes montrent que l'observation du pic représentant les variations de dTvap/dt en fonction du temps permet d'obtenir une détermination correcte du début du processus d'ébullition dans l'échantillon liquide présent dans le ballon de distillation environ 20 à 30 secondes avant le point IBP.

Le procédé conforme à l'invention permet ainsi de déterminer l'instant auquel doit être effectuée la dernière régulation conformément à la formule I.

A partir de cette dernière régulation, le procédé conforme à l'invention permet également d'effectuer la régulation de la température $T_{chauf}$ de l'élément chauffant à partir de la température $T_{liq}$ de l'échantillon liquide présent dans le ballon de distillation mesurée par le capteur infrarouge et de la température $T_{vap}$ des vapeurs évaporées mesurée par le thermomètre vapeur de façon à obtenir un point de distillation à 5 % correct dans les conditions imposées par la norme ASTM D 86.

Selon une autre caractéristique de l'invention, on a en effet pu établir que cette régulation pouvait être mise en oeuvre de façon satisfaisante conformément à la formule II ci-dessous :

$$\left(T_{chauf} - T_{liq}\right) =$$
$$A0 + A1.\exp(A2.T_{liq}) + A3.\left(T_{liq} - T_{vap}\right) \quad II$$

dans laquelle A0, A1, A2 et A3 sont des coefficients empiriques.

Dans le cas particulier de l'élément chauffant utilisé, ces coefficients avaient les valeurs suivantes : A0 = 243.3, A1 = 32.87, A2 = 6.796$^{e}$-3 et A3 = 0.53229.

En effet, la différence $T_{chauf}$ - $T_{liq}$ qui correspond essentiellement à l'énergie effectivement transmise à l'échantillon liquide présent dans le ballon de distillation peut être calculée à partir de deux valeurs, à savoir :

- d'une part la température de l'échantillon liquide mesurée par le capteur infrarouge, et
- d'autre part la différence entre la température de l'échantillon liquide mesurée par le capteur infrarouge et la température des vapeurs évaporées mesurée par le thermomètre vapeur.

La régulation de la température $T_{chauf}$ de l'élément chauffant conformément à la formule II peut être effectuée à partir du moment où les vapeurs évaporées commencent à monter dans la colonne du ballon de distillation.

Cette régulation peut être renouvelée toutes les 1 à 2 secondes compte tenu des modifications rapides de la température $T_{liq}$ de l'échantillon liquide présent dans le ballon de distillation et de la température $T_{vap}$ des vapeurs évaporées.

La régulation conformément à la formule II peut être arrêtée après récupération de 2 à 3 ml d'échantillon dans le cylindre collecteur et il est ensuite possible d'appliquer l'algorithme usuel de la régulation.

Selon une autre caractéristique de l'invention, on détermine par calcul l'instant pour lequel il ne reste plus que 5 % de l'échantillon à analyser dans le ballon de distillation en tenant compte des pertes et du volume du résidu en fin de distillation prévus, et on régule la température $T_{chauf}$ de l'élément chauffant entre l'instant ainsi déterminé et le point d'ébullition finale (FBP).

On a pu établir à partir de tests effectués sur des échantillons de produits commerciaux que pour satisfaire aux exigences de la norme AFTM D86, il s'avère nécessaire d'effectuer un ajustement final de la régulation de la température de l'élément chauffant à partir de l'instant pour lequel il ne reste plus que 5 ml de l'échantillon à analyser dans le ballon de distillation.

En effet, et selon les cas, à partir de cet instant, l'énergie calorifique transmise à l'échantillon liquide peut être conservée à sa valeur précédente ou doit être quelque peu diminuée ou augmentée.

Cette nécessité est liée à d'éventuelles modifications rapides de la dynamique de la distillation à la fin de celle-ci qui peuvent entraîner des variations brusques de la température et une augmentation de l'énergie nécessaire pour chauffer et évaporer l'échantillon liquide encore présent dans le ballon de distillation.

Les variations nécessaires de l'énergie calorifique transmise à l'échantillon liquide sont fonction de la pente de distillation pour les derniers 5 ml d'échantillon et du type d'échantillon comme précisé dans le tableau ci-dessous :

| Pente ($\Delta c/\Delta v$) pour les derniers 5 ml d'échantillon | Type d'échantillon | Ajustement nécessaire du chauffage de l'échantillon |
|---|---|---|
| similaire à la pente moyenne pour l'échantillon | Composés purs et quasi purs, produit de type éther, naphta de coupe étroite, solvants, etc... | Le chauffage peut être un peu diminué pour obtenir une extrémité fiable de la courbe de distillation pour la détermination du point FBP |
| un peu supérieure à la pente moyenne de l'échantillon | Coupes de distillation directe et la majorité des produits commerciaux habituels | Le chauffage peut être stabilisé au niveau de sa dernière valeur |
| nettement supérieure à la pente moyenne de l'échantillon | Produits commerciaux contaminés par des composés à point d'ébullition élevé, certains produits après raffinage secondaire | Le chauffage doit être augmenté d'autant plus que la pente est supérieure à la pente moyenne de l'échantillon |

Il est par suite nécessaire de pouvoir déterminer l'instant pour lequel il ne reste plus que 5 % de l'échantillon à analyser dans le ballon de distillation.

L'invention permet d'effectuer une telle détermination grâce à la prévision du volume du résidu en fin de distillation et des pertes.

Selon l'invention, on a effectué des essais de distillation selon la norme ASTM D86 sur des échantillons d'essence plus ou moins contaminés avec du carburant Diesel et déterminé les pentes moyennes de distillation dans la plage d'évaporation de 10 à 90 % du volume d'échantillon, et les pentes de distillation à l'instant où il ne reste plus que 5 ml d'échantillon dans le ballon de distillation ainsi que l'ajustement final nécessaire de l'énergie calorifique transmise à cet échantillon.

Les résultats ainsi obtenus sont rassemblés dans le tableau ci-dessous :

| Echantillon | Dérivée seconde $d^2T/dV^2$ | Ajustement nécessaire de l'énergie calorifique |
|---|---|---|
| Essence noncontam. | 0.058 | 0 |
| Essence + 1 % Diesel | 0.062 | 0 %...25 % |
| Essence+ 3 % Diesel | 0.292 | 25 %...50 % |
| Essence + 7 % Diesel | 0.799 | 25 %...50 % |
| Essence + 15 % Diesel | -0.056 | 0 %...25 % |

On a ainsi pu établir que l'ajustement nécessaire de l'énergie calorifique transmise à l'échantillon à l'instant où il ne restait plus que 5 ml d'échantillon dans le ballon de distillation était en corrélation avec la dérivée seconde $d^2T/dV^2$ des courbes de distillation peu avant cet instant.

Lors des essais de distillation susmentionnés, on a en particulier pu établir que la courbe représentant les variations de $d^2T/dV^2$ à l'instant pour lequel 92 % de l'échantillon avaient été évaporés en fonction du pourcentage de contamination de cet échantillon par du carburant Diesel présentait un maximum compris entre 3 et 10 % de contamination qui correspondait à la plage pour laquelle l'ajustement nécessaire de l'énergie calorifique était maximum.

Ce résultat est de nature à prouver que la dérivée seconde des courbes de distillation peut être utilisée pour permettre de prévoir l'ajustement final nécessaire de l'énergie calorifique transmise à l'échantillon en fin de distillation et par suite d'effectuer automatiquement cet ajustement.

Conformément à l'invention, pour prévoir le volume du résidu, on considère par hypothèse d'une part que, à l'instant du point d'ébullition final FBP, il ne reste plus de liquide au fond du ballon de distillation, et d'autre part, que le volume du résidu est constitué de deux parties, à savoir le volume $V_{Residu}^{Vap}$ de liquide condensé dans le ballon à partir des vapeurs évaporées et le volume $V_{Residu}^{Reflux}$ de liquide ayant reflué sur les parois du ballon.

En d'autres termes, le volume $V_{Residu}$ du résidu correspond à la somme suivante :

$$V_{\text{Residu}} = V_{\text{Residu}}^{\text{Vap}} + V_{\text{Residu}}^{\text{Reflux}}$$

Selon l'invention, on a ainsi constaté que le volume $V_{\text{Residu}}$ du résidu pouvait être représenté par l'équation III ci-dessous :

$$V_{\text{Residu}} = \left[ \frac{165 \cdot mm \cdot 273}{22.4 \cdot \rho \cdot \left(273 + T_{FBP}^{Prevu}\right)} \right] + \left[ C \cdot T_{FBP}^{Prevu} + D \right] \qquad (III)$$

dans laquelle :

- 165 représente le volume en millilitres d'un ballon de distillation correspondant à la norme AFTM D86,
- mm représente la masse moléculaire moyenne de la dernière partie d'échantillon évaporée,
- p représente la densité de la dernière partie d'échantillon évaporée, et
- $T_{FBP}^{\text{Pr}evu}$ représente la température prévue du point d'ébullition final FBP,
- C et D sont des coefficients empiriques obtenus en utilisant les particularités du ballon de distillation de la méthode D86.

La température $T_{FBP}^{\text{Pr}evu}$ peut être obtenue par extrapolation à partir des températures $T_{85\,\%}$ et $T_{90\,\%}$ pour lesquelles 85 % et 90 % du volume de l'échantillon ont été évaporés.

Les valeurs de mm et de p peuvent être calculées en utilisant des équations empiriques bien connues de l'homme du métier à partir de la température $T_{FBP}^{\text{Pr}evu}$ .

Selon l'invention, pour prévoir les pertes, on a considéré que lors d'une distillation mise en oeuvre conformément à la norme ASTM D 86, les pertes étaient directement liées à la pression de vapeur selon Reid (RVP) par la relation :

$$\text{Perte} = 0{,}0142 \; RVP \; (kPa)$$

Or, la pression de vapeur RVP peut être calculée en temps réel avant l'achèvement de la distillation (méthode normalisée utilisant la partie déjà distillée).

Des résultats expérimentaux ont permis de confirmer cette corrélation.

## Revendications

**1.** Procédé de distillation discontinue automatique d'échantillons liquides, en particulier d'échantillons de produits pétroliers sous prèssion atmosphérique dans un appareil de distillation de laboratoire permettant d'obtenir des caractéristiques, en particulier des tables ou des courbes de distillation dans des conditions spécifiées par une norme qui impose le classement d'un échantillon à analyser dans un groupe défini par celle-ci, et pour chaque groupe d'échantillons, des paramètres de distillation comprenant le temps écoulé entre le début du chauffage et le point d'ébullition initiale (IBP), le point de distillation à 5 % et le point pour lequel il ne reste que 5 % de l'échantillon dans le ballon de distillation, cet appareil de distillation renfermant :

- un élément chauffant,
- un ballon de distillation dont le col peut être fermé par un bouchon étanche et peut être connecté à un condenseur,
- un cylindre collecteur permettant de recueillir le condensât et équipé d'organes de mesure de la quantité de condensât ainsi recueillie en fonction du temps,
- des organes de mesure de la température des vapeurs évaporées, et
- des moyens de commande et de régulation permettant de commander et de faire varier dans le temps une grandeur de fonctionnement de l'élément chauffant, notamment la température ou la puissance électrique de cet élément de manière à obtenir des caractéristiques de distillation dans les conditions spécifiées par la norme,

selon lequel

- on introduit une quantité prédéfinie d'un échantillon à analyser dans le ballon de distillation,
- on positionne ce ballon de distillation sur l'élément chauffant, on le ferme et on le branche sur le condenseur,
- on classe l'échantillon à analyser dans un groupe défini par la norme, et
- on lance la distillation de l'échantillon à analyser en mesurant constamment la quantité de condensât recueillie dans le cylindre collecteur et la température des vapeurs évaporées, et
- on transmet les valeurs ainsi mesurées aux moyens de commande et de régulation de manière à leur permettre de commander en retour la température $T_{chauf}$ de l'élément chauffant,

procédé **caractérisé en ce qu'**

- on équipe le ballon de distillation d'organes de mesure par radiation de la température de l'échantillon liquide présent dans celui-ci, sans contact direct avec cet échantillon liquide,
- on mesure constamment cette température et on transmet la valeur ainsi mesurée aux moyens de commande et de régulation de sorte que :

- avant le point IBP, ces moyens régulent la température $T_{chauf}$ de l'élément chauffant en utilisant en tant que grandeur de régulation, la température $T_{liq}$ de l'échantillon liquide présent dans le ballon de distillation, conformément à la formule :

$$ - \quad \left(T_{chauf} - T_{liq}\right) = A \left(\frac{dTliq}{dt}\right)^{0,2513} $$

dans laquelle dTliq/dt représente les variations de la température de l'échantillon liquide en fonction du temps,
- entre le point IBP et le point de distillation à 5 %, ces moyens régulent la température $T_{chauf}$ de l'élément chauffant en utilisant en tant que grandeur de régulation, la température $T_{liq}$ de l'échantillon liquide présent dans le ballon de distillation ainsi que la température $T_{vap}$ des vapeurs évaporées mesurée par le thermomètre vapeur conformément à la formule :

$$ - \quad (T_{chauf} - T_{liq}) = $$
$$ - \quad A0 + A1.exp(A2.T_{liq}) + A3.(T_{liq} - T_{vap}) $$

dans laquelle A0, A1, A2 et A3 sont des coefficients empiriques, et
- après le point de distillation à 5 %, ces moyens régulent la température $T_{chauf}$ de l'élément chauffant de manière classique en utilisant uniquement en tant que grandeur de régulation, la température $T_{vap}$ des vapeurs évaporées,

de manière à obtenir directement et automatiquement des caractéristiques de distillation dans des conditions spécifiées par la norme.

2. Procédé selon la revendication 1,
**caractérisé en ce qu'**
on détermine par calcul l'instant pour lequel il ne reste plus que 5 % de l'échantillon à analyser dans le ballon de distillation en tenant compte des pertes et du volume du résidu en fin de distillation prévus, et on régule la température $T_{chauf}$ de l'élément chauffant entre l'instant ainsi déterminé et le point d'ébullition finale (FBP).

3. Procédé selon la revendication 1
**caractérisé en ce qu'**
on mesure la température de l'échantillon liquide présent dans le ballon de distillation au moyen d'un capteur infrarouge.

4. Procédé selon la revendication 3,
**caractérisé en ce qu'**
on interpose entre l'élément chauffant et le ballon de distillation une plaque isolante perforée imperméable au rayonnement infrarouge.

**5.** Procédé selon la revendication 4,
**caractérisé en ce que**
la plaque isolante imperméable au rayonnement infrarouge est réalisée en une céramique à base de silicate de calcium synthétique comprimée.

**6.** Procédé selon l'une quelconque des revendications 1 à 5,
**caractérisé en ce que**
la norme est la norme ASTM D 86.


**Patentansprüche**

**1.** Verfahren zur automatischen, diskontinuierlichen Destillation flüssiger Proben, insbesondere Proben von Erdölprodukten unter Atmosphärendruck in einer Labordestillationsvorrichtung, die es ermöglicht, Kenndaten, insbesondere Destillationstabellen oder -kurven unter Bedingungen, die in einer Norm spezifiziert sind, welche eine Einordnung einer zu analysierenden Probe in eine durch diese definierte Gruppe vorschreibt, und für jede Gruppe von Proben, Destillationsparameter, die die vergangene Zeit zwischen dem Anfang des Erhitzens und dem Anfangssiedepunkt (IBP), dem Destillationspunkt bei 5 % und dem Punkt, bei dem nur 5 % der Probe in dem Destillationskolben übrig bleiben, umfassen, zu erhalten, wobei die Destillationsvorrichtung umfasst:

- ein Heizelement,
- einen Destillationskolben, dessen Hals mit einem Dichtstopfen geschlossen und mit einem Kondensator verbunden werden kann,
- einen Sammelzylinder, der es ermöglicht, das Kondensat zu sammeln, und der mit Messorganen zur Messung der so gesammelten Menge von Kondensat abhängig von der Zeit ausgestattet ist,
- Messorgane zur Messung der Temperatur der verdampften Dämpfe, und
- Steuerungs- und Regelungsmittel, die es ermöglichen, eine Größe der Funktionsweise des Heizelements, insbesondere die Temperatur oder die elektrische Leistung dieses Elements, zu steuern und mit der Zeit zu variieren, um Destillationskenndaten unter den durch die Norm spezifizierten Bedingungen zu erhalten,

wobei

- eine vorbestimmte Menge einer zu analysierenden Probe in den Destillationskolben eingeführt wird,
- der Destillationskolben auf das Heizelement positioniert, geschlossen und an den Kondensator angeschlossen wird,
- die zu analysierende Probe einer durch die Norm definierten Gruppe zugeordnet wird,
- die Destillation der zu analysierenden Probe gestartet wird, indem die Menge von in dem Sammelzylinder gesammeltem Kondensat und die Temperatur der verdampften Dämpfe ständig gemessen werden, und
- die so gemessenen Werte an die Steuerungs- und Regelungsmittel übertragen werden, damit sie dazu ermöglicht werden, die Temperatur $T_{chau}f$ des Heizelements im Gegenzug zu steuern,

wobei das Verfahren **dadurch gekennzeichnet ist, dass**:

- der Destillationskolben mit Messorganen zur Messung durch Strahlung der Temperatur der flüssigen Probe, die darin vorliegt, ohne direkten Kontakt mit dieser flüssigen Probe, ausgestattet wird,
- diese Temperatur ständig gemessen wird und der so gemessene Wert an die Steuerungs- und Regelungsmittel übertragen wird, so dass:

- vor dem IBP Punkt, diese Mittel die Temperatur $T_{chau}f$ des Heizelements regeln, indem als Regelungsgröße die Temperatur $T_{liq}$ der flüssigen Probe, die in dem Destillationskolben vorliegt, verwendet wird, nach der Formel:

$$- \left(T_{chauf} - T_{liq}\right) = A \left(\frac{dT_{liq}}{dt}\right)^{0,2513}$$

wobei $dT_{liq}/dt$ die Variationen der Temperatur der flüssigen Probe abhängig von der Zeit repräsentiert,
- zwischen dem IBP Punkt und dem Destillationspunkt bei 5 %, diese Mittel die Temperatur $T_{chau}$f des Heizelements regeln, indem als Regelungsgröße die Temperatur $T_{liq}$ der flüssigen Probe, die in dem Destillationskolben vorliegt, sowie die Temperatur $T_{vap}$ der verdampften Dämpfe, die durch das Dampfthermometer gemessen wird, verwendet werden, nach der Formel:

$$- (T_{chauf} - T_{liq}) =$$

$$- A0 + A1.\exp(A2.T_{liq}) + A3.(T_{liq} - T_{vap})$$

wobei A0, A1, A2 und A3 empirische Koeffizienten sind, und
- nach dem Destillationspunkt bei 5 % diese Mittel die Temperatur $T_{chau}$f des Heizelements auf klassische Weise regeln, indem als Regelungsgröße ausschließlich die Temperatur $T_{vap}$ der verdampften Dämpfe verwendet wird,

um unmittelbar und automatisch Destillationskenndaten unter den durch die Norm spezifizierten Bedingungen zu erhalten.

**2.** Verfahren nach Anspruch 1,
**dadurch gekennzeichnet, dass**
der Zeitpunkt, an dem nur 5 % der zu analysierenden Probe in dem Destillationskolben übrig bleiben, durch Berechnung bestimmt wird, indem die vorgesehenen Verluste und das vorgesehene Volumen des Rückstands am Ende der Destillation berücksichtigt werden, und die Temperatur $T_{chauf}$ des Heizelements zwischen dem so bestimmten Zeitpunkt und dem Endsiedepunkt (FBP) geregelt wird.

**3.** Verfahren nach Anspruch 1,
**dadurch gekennzeichnet, dass**
die Temperatur der flüssigen Probe, die in dem Destillationskolben vorliegt, mittels eines Infrarotsensors gemessen wird.

**4.** Verfahren nach Anspruch 3,
**dadurch gekennzeichnet, dass**
zwischen das Heizelement und den Destillationskolben eine gelochte, für Infrarotstrahlung undurchlässige Isolierplatte gelegt wird.

**5.** Verfahren nach Anspruch 4,
**dadurch gekennzeichnet, dass**
die für Infrarotstrahlung undurchlässige Isolierplatte aus einer Keramik auf Grundlage von zusammengepresstem, synthetischem Calciumsilicat hergestellt wird.

**6.** Verfahren nach einem der Ansprüche 1 bis 5,
**dadurch gekennzeichnet, dass**
die Norm die Norm ASTM D 86 ist.

## Claims

**1.** Method for automatic discontinuous distillation of liquid samples, in particular samples of petroleum products, under atmospheric pressure in a laboratory distillation apparatus that makes it possible to obtain characteristics, in particular distillation tables or curves, in conditions specified by a standard that requires the classification of a sample to be analysed into a group defined by said standard, and, for each group of samples, distillation parameters including the time that has lapsed between the start of the heating and the initial boiling point (IBP), the 5% distillation point and the point at which there remains only 5% of the sample in the distillation balloon, said distillation apparatus comprising:

- a heating element,
- a distillation balloon of which the neck can be closed by a sealing plug and can be connected to a condenser,

- a collection cylinder that makes it possible to receive the condensate and is provided with devices for measuring the quantity of the condensate received in this manner as a function of time,
- devices for measuring the temperature of the evaporated vapours, and
- control and adjustment means which make it possible to control and to change, over time, an operating variable of the heating element, in particular the temperature or electrical power of said element, in order to obtain distillation characteristics in the conditions specified by the standard,

according to which method

- a predetermined quantity of a sample to be analysed is introduced into the distillation balloon,
- said distillation balloon is positioned on the heating element, is closed and is connected to the condenser,
- the sample to be analysed is classified into a group defined by the standard, and
- the distillation of the sample to be analysed is started by constantly measuring the quantity of condensate received in the collection cylinder and the temperature of the evaporated vapours, and
- the values measured in this manner are transmitted to the control and adjustment means in order for said means to be able to control the temperature $T_{chau}f$ of the heating element in response thereto,

said method being **characterised in that**

- the distillation balloon is provided with devices for measuring, by radiation, the temperature of the liquid sample present in said balloon, without any direct contact with said liquid sample,
- said temperature is measured constantly and the value measured in this way is transmitted to the control and adjustment means such that:

- before the IBP, said means adjust the temperature $T_{chau}f$ of the heating element by using the temperature $T_{liq}$ of the liquid sample present in the distillation balloon as a control variable, according to the following formula:

$$- \left( T_{chauf} - T_{liq} \right) = A \left( \frac{dTliq}{dt} \right)^{0.2513}$$

where $dTliq/dt$ represents the variations in the temperature of the liquid sample as a function of time,
- between the IBP and the 5% distillation point, said means adjust the temperature $T_{chau}f$ of the heating element by using the temperature $T_{liq}$ of the liquid sample present in the distillation balloon as well as the temperature $T_{vap}$ of the evaporated vapours that is measured by the vapour thermometer as a control variable, according to the following formula:

$$- (T_{chauf} - T_{liq}) =$$
$$- A0 + A1.exp(A2.T_{liq}) + A3.(T_{liq} - T_{vap})$$

where A0, A1, A2 and A3 are empirical coefficients, and
- after the 5% distillation point, said means adjust the temperature $T_{chau}f$ of the heating element in a conventional manner by using only the temperature $T_{vap}$ of the evaporated vapours as a control variable,

in order to directly and automatically obtain distillation characteristics in conditions specified by the standard.

2. Method according to claim 1, **characterised in that** the time at which there remains only 5% of the sample to be analysed in the distillation balloon is calculated by taking into account the predicted losses and predicted volume of the residue at the end of the distillation, and the temperature $T_{chauf}$ of the heating element is adjusted between the time determined in this manner and the final boiling point (FBP).

3. Method according to claim 1, **characterised in that** the temperature of the liquid sample present in the distillation balloon is measured by means of an infrared sensor.

4. Method according to claim 3, **characterised in that** a perforated insulation plate that is impermeable to infrared radiation is arranged between the heating element and the distillation balloon.

5. Method according to claim 4, **characterised in that** the insulation plate that is impermeable to infrared radiation is formed by a ceramic based on compressed synthetic calcium silicate.

6. Method according to any one of claims 1 to 5, **characterised in that** the standard is the ASTM D 86 standard.

ANNEXE 1

ANNEXE II

$$\left(T_{chauf} - T_{liquid}\right) = 650 \left(\frac{dTliq}{dt}\right)^{0.273}$$

Tchauf - Tliq, °C

dT/dt, °C/sec

EP 2 111 544 B1

ANNEXE III

Échantillon de carburant Diesel

Échantillon de carburant Diesel

ANNEXE IV

Échantillon d'essence

**EP 2 111 544 B1**

## RÉFÉRENCES CITÉES DANS LA DESCRIPTION

**Documents brevets cités dans la description**

- US 3364731 A